# NOUVEAU FASCICULE DE BREVET EUROPEEN

(11) **EP 0 284 506 B2**
(45) Date de publication et mention de la décision concernant l'opposition: **29.03.2000**
(45) Mention de la délivrance du brevet: 19.02.1992
(21) Numéro de dépôt: 88400679.2
(22) Date de dépôt: 17.02.1988
(51) Int. Cl.: A61F 13/00

(54) **Procédé de fabrication d'une bande de contention cohésive et les moyens de mise en oeuvre**
Verfahren und Vorrichtung zur Herstellung zusammenhängender elastischer Bandagen
Process and means for making a cohesive elastic bandage

(30) Priorité: 17.02.1987 FR 8703355
(43) Date de publication de la demande: 28.09.1988
(73) Titulaire: MOLYPHARM INDUSTRIES, 42340 Veauche (FR)
(72) Inventeur:
(74) Mandataire: Thivillier, Patrick

(56) Documents cités:
- WO-A-85/01660
- DE-C- 2 031 001
- DE-C- 2 730 277
- DE-C- 2 912 129
- FR-A- 815 283
- FR-A- 2 452 290
- US-A- 2 238 878
- US-A- 3 575 782
- US-A- 4 207 885
- Vliesstoffe, Prof. Lünenschloss, Thieme, Stuttgart 1982, page 189

## Description

L'objet de l'invention se rattache au secteur technique des bandages.

On connaît déjà des bandes de contention dites cohésives, c'est-à-dire susceptibles de n'adhérer qu'à elles-mêmes et non à la peau. Dans ce type de bandes, il convient de distinguer celles qui sont obtenues sur un support non tissé et celles qui sont obtenues sur un support tissé ou tricoté. Généralement, le caractère cohésif est obtenu par pulvérisation de fines goutellettes de latex.

Cet état de la technique peut être illustré par l'enseignement des documents WO-A-8501660 et FR-A-2452290.

Par le document WO-A-8501660, on connaît une bande de contention qui est recouverte sur la totalité de chacune de ses faces, d'une péllicule constituant une couche adhésive. Par définition, cette pellicule obture les interstices résultant de l'entrelacement des fils de chaîne et des fils de trame, de sorte que la bande ne peut pas respirer. En outre, le produit obtenu est très rigide.

Or, il est très important qu'une bande de contention soit suffisamment aérée, compte-tenu de son application médicale pour permettre à la peau de respirer et d'éviter tout phénomène de mascération.

Par le document FR-A-2452290, on connaît une bande de contention où l'adhésif est appliqué et réparti de manière uniforme sur les deux surfaces de la structure plane du bandage. Cette technique d'enduction de l'adhésif nécessite de travailler le bandage dans un plan vertical pour soumettre chacune des faces de la bande, à une projection de l'adhésif. Il apparait donc qu'au fur et à mesure de l'avance de la bande, cette dernière est soumise à un certain poids créant un effet d'étirement non contrôlable.

Or, le fait de connaître très précisemment le degré d'allongement de la bande de contention, s'avère très important dans la pratique, au niveau de la prescription médicale. En fonction du traumatisme à soigner, il est préscrit une bande ayant un certain degré d'allongement, correspondant à la pression que l'on souhaite exercer. De telles dispositions s'avèrent particulièrement importantes, dans le domaine de la phlébologie.

Le problème que se propose de résoudre l'invention est d'avoir une bande parfaitement aérée et de pouvoir connaître, d'une manière précise, son allongement après les différentes étapes de traitement, notamment après son enduction, pour contrôler de manière continue, le degré d'allongement de la bande.

On rappelle de manière connue, qu'un procédé de fabrication d'une bande de contention cohésive, comprend les principales étapes suivantes voir par exemple FR-A-2 452 290 :
- on soumet ladite bande à une opération de rétraction pour lui donner un allongement compris entre 110 et 140 %,
- on conditionne la bande en rouleau,
- on asservit le rouleau de conditionnement à un organe de commande pour lui assurer un déroulement positif correspondant à l'allongement initial compris entre 110 et 140 %, puis, d'une manière successive et continue,
- on soumet la bande, à la sortie du rouleau de conditionnement à une opération de rétraction pour lui conférer un allongement compris entre 90 et 110 %.

Selon l'invention, pour résoudre le problème posé de contrôler le degré d'allongement de la bande, le procédé est caractérisé en ce que, on entraine linéairement la bande dans un plan horizontal au moyen d'un tapis roulant au niveau d'une cabine de pulvérisation et de séchage
- on soumet l'une seulement des faces de la bande à une projection d'une émulsion aqueuse de gomme naturelle,
- on soumet cette face à une opération de séchage.
- on conditionne la bande sur un rouleau de réception de sorte que la face émulsionnée soit située du côté interne,
- on met le rouleau de réception en lieu et place du rouleau de conditionnement de départ pour acheminer, la bande dans les conditions indiquées précédemment pour traiter dans un deuxième temps,
- l'autre face de la bande en la soumettant successivement à une projection d'une émulsion aqueuse de gomme naturelle et à une opération de séchage pour obtenir la pluralité de bouclettes de caoutchouc.

Avantageusement, le problème posé est résolu en ce que l'on tisse ou l'on tricote la bande selon des lés de grande largeur et on déplace transversalement et alternativement les moyens de projection de l'émulsion aqueuse de gomme naturelle, selon toute la largeur de ladite bande et au fur et à mesure de son défilement.

Compte-tenu de ce procédé, la bande obtenue est particulièrement bien adaptée dans le domaine de la phlébologie et les traumatismes sportifs.

Pour la mise en oeuvre du procédé, et compte-tenu du problème posé à résoudre, les moyens comprennent un organe de commande pour l'entraînement du rouleau de conditionnement en vue de son déroulement selon un allongement compris entre 110 et 140 % ; un donneur positif pour conférer à la bande un allongement compris entre 90 et 110 % ; un tapis roulant pour entrainer linéairement la bande dans un plan horizontal au niveau d'une cabine de pulvérisation comprenant au moins une tête pour la projection de l'emulsion aqueuse de gomme naturelle, ladite tête étant susceptible d'être deplacée transversalement et alternativement selon toute la largeur de la bande d'une cabine de séchage.

L'invention est exposée ci-après plus en détail à l'aide des dessins qui représentent seulement un mode d'exécution :
- la figure 1 est une vue à caractère purement schématique illustrant l'installation pour la mise en oeuvre du procédé,
- la figure 2 est une vue en coupe transversale considérée selon la ligne 2.2. de la figure 1,
- les figures 3 et 4 sont, à grande échelle, des vues partielles en coupe longitudinale de la bande,
- la figure 5 montre un échantillon d'un exemple de réalisation de la bande cohésive.

Afin de rendre plus concret l'objet de l'invention, on le décrit maintenant d'une manière non limitative en se référant aux exemples de réalisation des figures des dessins.

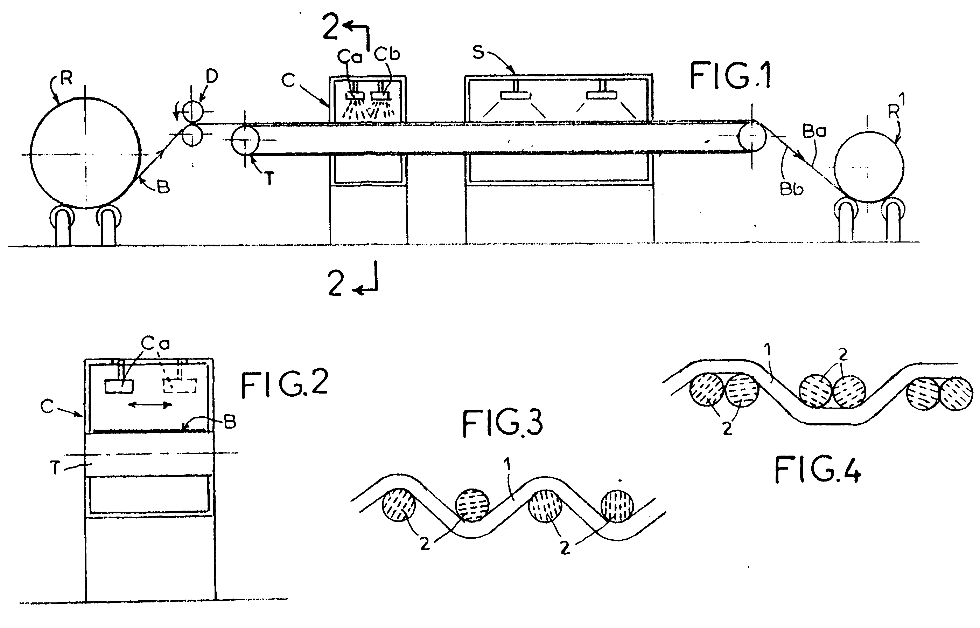

La bande est obtenue à partir d'un tissu résultant de l'entrelacement des fils de chaîne (1) et des fils de trame (2) selon une armure donnée. Ce tissage peut être classique, c'est-à-dire entrelacement successif d'un fil de chaîne et d'un fil de trame (figure 3) ou bien ce tissage peut être exécuté sur un métier à crochet en réalisant un entrelacement successif d'un fil de chaîne (1) et de deux fils de trame (2) (figure 4).

Plusieurs modes de tissage et de tricotage peuvent être prévus quant à la nature des fils les composant, à savoir:
- chaîne composée de fils extensibles et trame en fils non extensibles,
- chaîne et trame composées de fils extensibles,
- chaîne composée en alternance de fils extensibles et inextensibles, trame en fils non extensibles,
- chaîne composée en alternance de fils extensibles et inextensibles, trame en fils extensibles.

A titre d'exemple indicatif nullement limitatif, les fils composant le tissu, sont du coton, de la viscose et de l'élasthane, dans les proportions suivantes :
- - coton: : 21 %
- - viscose: : 78,2 %
- - élasthane: : 0,8 %
La figure 1 illustre un exemple d'installation pour la mise en oeuvre du procédé.

Après tissage ou tricotage, la bande (B) est soumise à une rétraction préalable à la vapeur pour lui donner un allongement compris entre 110 et 140 %. La bande est ensuite conditionnée en rouleau.

Le rouleau de conditionnement (R) de la bande est asservi à un organe de commande conformé pour assurer à ladite bande un déroulement positif correspondant à l'allongement initial compris entre 110 et 140 %. A la sortie du rouleau de conditionnement (R), la bande (B) est soumise à un donneur positif (D) pour lui conférer un allongement compris entre 90 et 110 %. La bande est alors acheminée, au moyen d'un tapis roulant (T), dans une cabine de pulvérisation (C) pour soumettre l'une des faces seulement de la bande, à une projection d'une émulsion aqueuse de gomme naturelle du type LATEX liquide,ou autre masse liquide adhésive de caoutchouc. A la sortie de la cabine de pulvérisation, la face émulsionnée (Ba) passe dans une cabine de séchage (S) puis est conditionnée sur un rouleau de réception (R1) de sorte que ladite face émulsionnée soit située du côté interne.

Le rouleau de réception (R1) est alors positionné sur le mandrin support du rouleau de conditionnement asservi au donneur positif (D), pour acheminer dans les conditions indiquées précédemment, la bande (B) dans la cabine de pulvérisation afin de soumettre l'autre face (Bb) à la projection de Latex, puis à l'opération de séchage. On obtient donc une pluralité de bouclettes de LATEX susceptibles de s'enchevêtrer lorsque deux parties de la bande sont mises en contact et en pression en lui conférant ainsi le caractère cohésif recherché.

A noter que la cabine de pulvérisation met en oeuvre des moyens parfaitement connus et conformés pour assurer une répartition constante et uniforme de l'émulsion aqueuse de gomme naturelle sur chacune des faces de la bande.

D'une manière préférée, cette pulvérisation s'effectue au moyen de deux buses d'injection jointives (Ca) et (Cb). De même, étant donné que la bande, pour des raisons évidentes d'économie, est traitée selon de grandes largeurs, les buses d'injection sont assujetties à un support pour être déplacées transversalement et alternativement selon toute la largeur de la bande, au fur et à mesure de son déplacement.

A la sortie de l'installation, après les différents traitements et étapes, la bande cohésive obtenue a un allongement compris entre 70 et 90 %. Les lisières sont supprimées pour autoriser un déchirement transversal de la bande de façon manuelle, sans l'emploi d'un outil de coupe quelconque.

De plus, l'imprégnation ainsi obtenue par pulvérisation d'une émulsion aqueuse de gomme naturelle, facilite le déchirement manuel de la boucle. Il est entendu que le lé de la bande est découpé selon différentes largeurs correspondant aux largeurs de bandes normalisées.

De même, pour assurer un meilleur accrochage du latex sur la bande, on prévoit d'exécuter toutes les trames en coton.

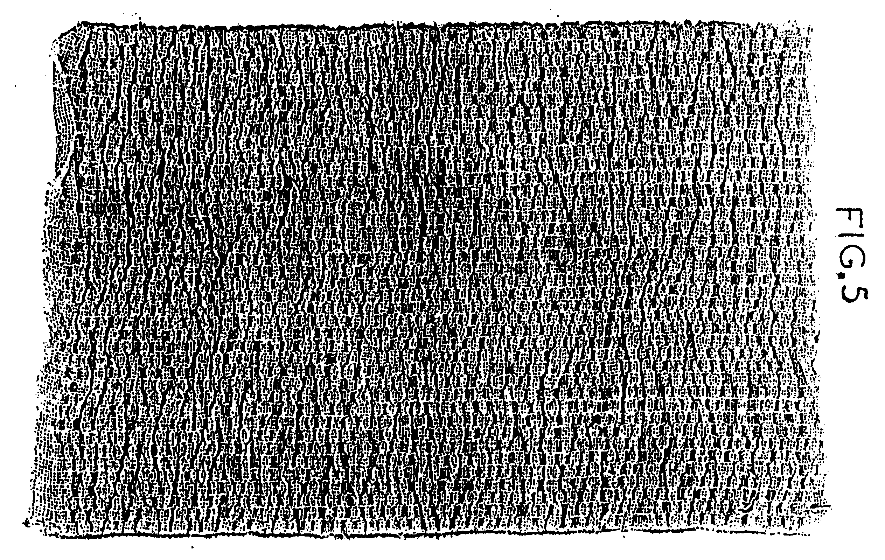

On renvoie à la figure 5 des dessins qui montre un échantillon de la bande cohésive selon l'invention. Il est bien évident que l'aspect de cette bande peut être différent et présenter un effet plus ou moins aéré en fonction de l'armure du tissage ou tricotage de base.

La bande de contention cohésive résultant du procédé, conserve ses qualités thérapeutiques et est particulièrement recommandée dans le domaine de la phlébologie et des traumatismes sportifs.

## Revendications

1. Procédé de fabrication d'une bande de contention cohésive obtenue par tissage ou tricotage et présentant une pluralité de bouclettes de caoutchouc, susceptibles de s'enchevêtrer lorsque deux parties de la bande sont mises en contact et en pression, procédé selon lequel :
- on soumet ladite bande à une opération de rétraction pour lui donner un allongement compris entre 110 et 140 %;
- on conditionne la bande en rouleau ;
- on asservit le rouleau de conditionnement à un organe de commande pour lui assurer un déroulement positif correspondant à l'allongement initial compris entre 110 et 140 % puis, d'une manière successive et continue,
- on soumet la bande, à la sortie du rouleau de conditionnement à une opération de rétraction pour lui conférer un allongement compris entre 90 et 110 %,
- on entraine linéairement la bande dans un plan horizontal au moyen d'un tapis roulant, au niveau d'un cabine de pulvérisation et de séchage,
- on soumet, dans un premier temps, l'une seulement des faces de la bande à une projection d'une émulsion aqueuse de gomme naturelle,
- on soumet cette face à une opération de séchage,
- on conditionne la bande sur un rouleau de réception de sorte que la face émulsionnée soit située du côté interne,
- on met le rouleau de réception en lieu et place du rouleau de conditionnement de départ pour acheminer la bande, dans les conditions indiquées precédement pour traiter, dans un deuxième temps, l'autre face de ladite bande en la soumettant successivement à une projection d'une émulsion aqueuse de gomme naturelle et à une opération de séchage pour obtenir la pluralité de bouclette de caoutchouc.

2. Procédé selon la revendication 1, caractérisé en ce que l'on tisse ou l'on tricote la bande selon des lés de grande largeur et on déplace transversalement et alternativement les moyens de projection de l'émulsion aqueuse de gomme naturelle, selon toute la largeur de ladite bande et au fur et à mesure de son défilement.

3. Procédé selon la revendication 1, caractérisé en ce que l'on supprime les lisières pour permettre un déchirement transversal de la bande.

4. Moyens de mise en oeuvre du procédé selon la revendication 1, caractérisés en ce qu'ils comprennent un organe de commande pour l'entraînement du rouleau de conditionnement en vue de son déroulement selon un allongement compris entre 110 et 140 % ; un donneur positif pour conférer à la bande un allongement compris entre 90 et 110 % ; un tapis roulant pour entrainer linéarement la bande dans un plan horizontal au niveau d'une cabine de pulvérisation et de séchage; la cabine de pulvérisation comprenant au moins une tête pour la projection de l'émulsion aqueuse de gomme naturelle ladite tête étant susceptible d'être déplacée transversalement et alternativement selon toute la largeur de la bande une cabine de séchage.

## Claims

1. Process for manufacturing a cohesive support web obtained by weaving or knitting and having a plurality of small rubber loops capable of entangling when two parts of the web are brought into contact and put under pressure, in which process :
- the said web is subjected to a retraction operation in order to give it an elongation of between 110 and 140%,
- the web is packaged as a roll,
- the pay-out roll is slaved to a drive member in order to ensure that it undergoes a positive unwinding corresponding to the initial elongation of between 110 and 140% and then, in a successive and continuous manner,
- the web, on leaving the pay-out roll, is subjected to a retraction operation in order to confer on it an elongation of between 90 and 110%,
- the web is driven linearly in a horizontal plane, by means of a conveyor belt, through a spray and drying booth,
- firstly, only one of the faces of the web is sprayed with an aqueous emulsion of natural gum,
- this face is subjected to a drying operation,
- the web is packaged on a take-up roll so that the emulsion-coated face is located on the internal side,
- the take-up roll is put in the place of the initial pay-out roll in order to convey the web, under the abovementioned conditions, and secondly to treat the other face of the said web by successively subjecting it to a spray of an aqueous emulsion of natural gum and to a drying operation in order to obtain the plurality of small rubber loops.

2. Process according to Claim 1, characterized in that the web is woven or knitted as wide widths and the means for spraying the aqueous emulsion of natural gum are moved transversely and reciprocatingly over the entire width of the said web and as it runs progressively forwards.

3. Process according to Claim 1, characterized in that the selvedges are omitted in order to allow transverse tearing of the web.

4. Means for implementing the process according to Claim 1, characterized in that they comprise a drive member for driving the pay-out roll for the purpose of unwinding it with an elongation of between 110 and 140%; a positive feeder for conferring on the web an elongation of between 90 and 110%; a conveyor belt in order to drive the web linearly in a horizontal plane through a spray and drying booth, the spray booth including at least one head for spraying the aqueous emulsion of natural gum, the said head being capable of being moved transversely and reciprocatingly over the entire length of the web; a drying booth.

## Patentansprüche

1. Verfahren zur Herstellung eines gewebten oder gestrickten Haftkompressionsbandes mit mehreren Gummischlingen, die geeignet sind, sich zu verfangen, wenn zwei Teile des Bandes unter Druck miteinander in Kontakt gebracht werden,
bei dem :
- das Band einem Retraktionsvorgang ausgesetzt wird, um es zwischen 110 und 140% zu dehnen,
- das Band als Rolle konditioniert wird,
- die Konditionierrolle von einem Steuerorgan abhängig gemacht wird, um sie entsprechend der ursprünglichen Dehnung zwischen 110 und 140%, dann sukzessiv und kontinuierlich abzuwickeln,
- das Band am Ausgang der Konditionierrolle einem Retraktionsvorgang ausgesetzt wird, um es zwischen 90 und 110% zu dehnen,
- das Band linear in horizontaler Ebene mit einem Förderband zu einer Spritz- und Trockenkabine geführt wird,
- zuerst nur eine Seite des Bandes mit einer wässrigen Emulsion aus Naturgummi bespritzt wird,
- diese Seite getrocknet wird,
- das Band auf einer Aufnahmerolle so konditioniert wird, daß die emulgierte Seite innen zu liegen kommt,
- die Aufnahmerolle unter den zuvor genannten Bedingungen an Ort und Stelle der anfänglichen Konditionierrolle für den Bandtransport gesetzt wird, um in einem zweiten Schritt die andere Seite des Bandes zu behandeln, indem es nacheinander mit einer wässrigen Emulsion aus Naturgummi bespritzt und getrocknet wird, um die vielen Gummischlingen zu erzielen.

2. Verfahren nach Anspruch 1, dadurch gekennzeichnet, daß das Band in Bahnen großer Breite gewebt oder gestrickt wird und die Spritzeinrichtungen der wässrigen Emulsion aus Naturgummi in der ganzen Breite des Bandes und nach Maßgabe seines Vorbeilaufs in Querrichtung und alternierend verlagert werden.

3. Verfahren nach Anspruch 1, dadurch gekennzeichnet, daß die Randleisten beseitigt werden, um ein Querreißen des Bandes zu ermöglichen.

4. Einrichtungen für die Umsetzung des Verfahrens nach Anspruch 1, dadurch gekennzeichnet, daß sie folgende Teile umfassen: ein Steuerorgan für die Mitnahme der Konditionierrolle zwecks Abwicklung gemäß einer Dehnung zwischen 110 und 140 %; einen Geber, um das Band zwischen 90 und 110 % zu dehnen ; ein Förderband, um das Band linear in horizontaler Ebene zu einer Spritz- und Trockenkabine zu führen, wobei die Spritzkabine mindestens einen Kopf für das Spritzen der wässrigen Emulsion aus Naturgummi umfaßt und der Kopf geeignet ist, in ganzer Bandlänge quer und alternierend verlagert zu werden ; eine Trockenkabine.
